# EUROPÄISCHE PATENTSCHRIFT

(11) **EP 1 171 104 B1**
(45) Veröffentlichungstag und Bekanntmachung des Hinweises auf die Patenterteilung: **13.06.2007**
(21) Anmeldenummer: 00922615.0
(22) Anmeldetag: 07.04.2000
(51) Int. Cl.: A61K 9/70

(54) **TRANSDERMALES THERAPEUTISCHES SYSTEM MIT NEUTRALISIERTEN ACRYLHAFTKLEBERN**
TRANSDERMAL THERAPEUTIC SYSTEM WITH NEUTRALIZED ACRYLIC ADHESIVE PATCH
SYSTEME THERAPEUTIQUE TRANSDERMIQUE A ADHESIFS DE CONTACT ACRYLIQUES NEUTRALISES

(30) Priorität: 22.04.1999 DE 19918106
(43) Veröffentlichungstag der Anmeldung: 16.01.2002
(73) Patentinhaber: LTS LOHMANN Therapie-Systeme AG, 56626 Andernach (DE)
(72) Erfinder: BRACHT, Stefan, D-56299 Ochtendung (DE)
(74) Vertreter: Flaccus, Rolf-Dieter
(86) Internationale Anmeldenummer: PCT/EP2000/003112
(87) Internationale Veröffentlichungsnummer: WO 2000/064418

(56) Entgegenhaltungen:
- EP-A- 0 394 956
- EP-A- 0 446 636
- WO-A-99/49852
- US-A- 4 880 416

## Beschreibung

Die Erfindung betrifft die chemische Neutralisierung von haftklebenden Polymeren oder Copolymeren, die in ihrer Polymerkette Acrylsäure oder Methacrylsäure eingebaut enthalten.

Polymere auf der Basis von Acrylsäure bzw. Methacrylsäure und deren Estern nehmen eine besonders wichtige Position unter den Haftklebern ein, da sie nicht nur Gerüstbildner und häufig Hauptbestandteil einer haftklebenden Formulierung sind, sondern selbst haftklebende Eigenschaften besitzen. Darin unterscheiden sie sich z.B. fundamental von den Mischungen aus Natur- oder Synthesekautschuken (Gerüstbildner) mit natürlichen oder synthetischen Harzen (sogenannte Tackifier).

Haftklebern auf Polyacrylatbasis müssen keine niedermolekularen Bestandteile zugesetzt werden, damit sie ihre haftklebenden Eigenschaften erhalten.

Neben den vielfältigen technischen Einsatzmöglichkeiten macht die letztgenannte Eigenschaft die Polyacrylat-Haftkleber besonders attraktiv für die medizinische Verwendung an Mensch oder Tier. Die niedermolekularen Bestandteile, meist Harze, wie sie als klebrigmachende Zusätze zu Kautschuken erforderlich sind, können bei der Aufnahme über die Haut Reizungen und sogar allergische Reaktionen auslösen. Dieses Risiko entfällt bei Polyacrylaten weitgehend, weshalb sie in der medizinischen Anwendung auch als "hypoallergen" bezeichnet werden.

Polyacrylat-Haftkleber finden heute sehr breite Anwendung bei der Herstellung medizinischer Pflaster zur Wundbehandlung oder Fixierung bei medizinischen Eingriffen (Stichwort "Heftpflaster"). Darüberhinaus stellen sie die wichtigste Gruppe von Haftklebern dar, die für die Herstellung von transdermalen therapeutischen Systemen (TTS) verwendet werden.

Abgesehen von der guten Hautverträglichkeit ist dies durch folgende Eigenschaften bedingt:

Polyacrylate können in vielfältiger Weise aus einer großen Auswahl von Monomeren aufgebaut werden. Auf diesem Wege können die haftklebenden Eigenschaften der Polymere und ihre Affinität zu den zu beklebenden Flächen, z.B. der menschlichen Haut, in weiten Grenzen eingestellt werden. Dabei spielt insbesondere die chemische Natur der Seitenketten am Polyacrylatrückgrat eine entscheidende Rolle. Diese bestimmen nicht nur die Hyrophilie-Lipophilie-Balance innerhalb des Polymers und damit z.B. die mögliche Feuchtigkeitsaufnahme. Insbesondere läßt sich durch geeignete Seitenketten und deren Mischung die Kristallinität des Polymers herabsetzen. Eine Herabsetzung der Kristallinität und damit der Glasübergangstemperatur wirkt sich positiv auf die haftklebenden Eigenschaften des Polymers aus, indem die Fließfähigkeit und damit die rasche Benetzung von Oberflächen begünstigt wird.

Für die medizinische Anwendung in TTS spielt eine niedrige Glasübergangstemperatur eine besondere Rolle: Das Polymer bzw. seine Seitenketten sind im nicht-kristallinen Zustand besonders durchlässig für enthaltene pharmazeutische-Wirk- und Hilfsstoffe. Dies ist für die rasche Freisetzung am Applikationsort essentiell.

Polyacrylate weisen für die meisten pharmazeutischen Wirkstoffe eine hohe Löslichkeit auf. Diese ist typischerweise höher als in anderen für die TTS-Herstellung geeigneten Haftklebern, wie z.B. Naturkautschuk-Harz-Gemischen oder auch in Silikonhaftklebern.

Häufig können die erforderlichen Mengen eines Wirkstoffs überhaupt nur in Polyacrylaten gelöst und damit in der zur Freisetzung am besten geeigneten Form in ein TTS eingearbeitet werden.

Polyacrylate können bei Einpolymerisierung von unveresterter Acrylsäure oder Methacrylsäure an ihrer Kette freie Carboxylgruppen tragen. Diese Carboxylgruppen eignen sich, darüber nachträglich mehrere Polymerketten miteinander zu verknüpfen. Typische und in der Fachwelt allgemein bekannte Reagenzien sind Organo-Metall-Komplexe wie z.B. Aluminium- oder Titanylacetylacetonat. Diese bringen mehrwertige Kationen in das Polymer ein, die dann mit mehreren Carboxylgruppen auf verschiedenen Polymerketten gleichzeitig Verbindungen eingehen.

Auf diese Weise können lineare Polymerketten dreidimensional vernetzt werden. Typischerweise geschieht dies beim Erhitzen und Trocknen der entsprechenden Polymerlösung im Rahmen der Verarbeitung zum Endprodukt.

Andere Möglichkeiten der Quervernetzung ergeben sich durch Einstrahlung energiereicher Lichtquanten, z.B. UV-Strahlung, in Verbindung mit geeigneten Vernetzungsreagenzien.

Die Quervernetzung verhindert die Fließfähigkeit der Polymermasse unter Aufrechterhaltung einer im wesentlichen nur noch elastischen Verformbarkeit.

Bei Verzicht auf eine Quervernetzung kommt es typischerweise zu einem als "Kalter Fluß" bekannten, unerwünschten langsamen Fließen des Haftklebers unter jedweder äußeren Krafteinwirkung, im einfachsten Fall der Schwerkraft.

Bei der Applikation auf der Haut kann der kalte Fluß zu einem unerwünscht tiefen Eindringen in die Poren der Haut führen und damit die Wiederablösung erschweren bzw. schmerzhaft werden lassen. Auch hier bietet die Quervernetzbarkeit entsprechende Vorteile.

Sie ist damit eine der wichtigsten Eigenschaften der Polyacrylate.

Polyacrylate werden durch Polymerisation des Vinylrestes - der Acryl- bzw. Methacrylsäure erhalten. Dieser Mechanismus erlaubt sehr einfach den Einbau fremder Monomere (Nicht-(Meth)Acrylate), die ebenfalls einen ethylenisch ungesättigten Molekülteil enthalten. Dies sind z.B. Ethylen, Vinylacetat oder andere Ester des Vinylalkohols und insbesondere verschiedene Vinylpyrrolidone sowie auch Styrol und Crotamiton.

Im Bereich von medizinischen Haftklebern finden sich z.B. zahlreiche Mischpolymere mit Vinylpyrrolidonen. Damit lassen sich u.a. höhere Löslichkeiten bestimmter Wirkstoffe oder auch eine höhere Feuchtigkeitsaufnahme bzw. -toleranz am Applikationsort Haut einstellen.

In der Summe stellen die Polyacrylate nicht nur im Bereich technischer Anwendungen eine unverzichtbare Gruppe von Haftklebern dar.

Insbesondere in der medizinischen Anwendung nehmen sie wegen der Summe ihrer positiven Eigenschaften in Kombination mit der preisgünstigen Verfügbarkeit eine überragende Position ein.

Die vorliegende Erfindung betrifft die chemische Modifizierung von sauren Polyacrylathaftklebern. Unter sauren Polyacrylathaftklebern sind Polymere mit folgenden Eigenschaften zu verstehen:
- das Polymer hat bei Raumtemperatur haftklebende Eigenschaften.
- bezogen auf die mittlere Polymermasse handelt es sich bei mindestens 50% (w/w) davon um Monomere aus der Gruppe von Acryl- oder Methacrylsäure oder deren Esterderivaten.
- bezogen auf die mittlere Polymermasse handelt es sich bei mindestens 0.5% (w/w), höchstens aber 10% (w/w) davon um unveresterte Acryl- oder Methacrylsäuren.

Der Erfindung lag die Aufgabe zugrunde, die Wasseraufnahmefähigkeit sowie die Toleranz saurer Polyacrylathaftkleber gegenüber Feuchtigkeit allgemein zu erhöhen.

Dies ist speziell bei der medizinischen Verwendung an Mensch oder Tier wünschenswert, da die Haftkleber auf der Haut der ständigen Wasserdampfabgabe über die Haut sowie beim Menschen auch der Feuchtigkeit durch Schwitzen ausgesetzt sind. Häufig werden die haftklebenden Eigenschaften unter diesen Bedingungen erheblich verschlechtert, so daß sich ein medizinisches Pflaster vorzeitig von der Haut ablöst. Bereits beim Aufkleben eines medizinischen Pflasters auf feuchte Haut kann es bei herkömmlichen sauren Polyacrylathaftklebern zu Problemen kommen.

Noch in einem weiteren Zusammenhang ist das Aufnahmevermögen von Feuchtigkeit eine wünschenwerte Verbesserung:

Bei transdermalen therapeutischen Systemen ist in der Haftkleberschicht typischerweise ein pharmazeutischer Wirkstoff enthalten. Solche Wirkstoffe sind zumeist lipophile Substanzen, wie sie in Polyacrylaten häufig sehr gut löslich sind. Für die Freisetzung des Wirkstoffs am Applikationsort ist eine gute Löslichkeit in der Klebermatrix jedoch hinderlich. Die Aufnahme von Feuchtigkeit in die Haftklebermatrix am Applikationsort (Wasserdampf, Schweiß) kann die Löslichkeit lipophiler Wirkstoffe darin senken. Ein erhöhtes Aufnahmevermögen an Feuchtigkeit wirkt daher positiv auf die Freisetzung vieler lipophiler Wirkstoffe aus einem Polyacrylathaftkleber.

Der Erfindung lag weiterhin die Aufgabe zugrunde, die Wirkstoffreisetzung basischer Wirkstoffe aus sauren, haftklebenden Acrylatcopolymeren zu verbessern. Die Freisetzung basischer Wirkstoffe aus solchen Polymeren wird durch diechemische Säure-Base-Wechselwirkung häufig erheblich verringert bzw. verzögert, so daß diese Kombination für die Entwicklung eines TTS, welches eine möglichst hohe Abgaberate des Wirkstoffs an die Haut leisten soll, praktisch wenig sinnvoll ist.

Die gestellte Aufgabe wird erfindungsgemäß dadurch gelöst, daß die in sauren Polyacrylathaftklebern enthaltenen Carboxylgruppen ganz oder teilweise neutralisiert werden, indem sie in die Alkali- oder Erdalkalisalze umgewandelt werden. Als Reagenz dienen alkalische Verbindungen der Alkali- oder Erdalkalimetalle, vorzugsweise deren Hydroxide wie Natrium- oder Kaliumhydroxid.

Die entstehenden Polymersalze haben gemäß der Natur aller ionisch geladenen Moleküle oder Molekülteile ein hohes Bindungsvermögen für Wasser in Form von Hydrathüllen. Insbesondere die Gegenionen Natrium und Kalium vermögen reichlich Wasser in dieser Art zu binden.

Neutralisieren oder Neutralisierung heißt, die vorhandenen sauer reagierenden Carboxylgruppen durch Umsetzung mit einer Base zu versalzen. Neutralisation bedeutet die vollständige Versalzung aller sauren Gruppen in dieser Weise. Der Neutralisationsgrad in Prozent drückt aus, zu welchem Anteil die Versalzung gegenüber der theoretisch möglichen vollständigen Umsetzung erfolgt ist.

Ein Neutralisationsgrad von mehr als 100% sollte vermieden werden, da das System aus Alkalicarboxylat und überschüssigem Alkali keinen Puffer aufbaut, sondern ein stark alkalisches und damit chemisch zersetzendes Milieu bilden kann. Ein Neutralisationsgrad von mehr als 100% kann aber sinnvoll sein, wenn andere saure Komponenten in der Rezeptur enthalten sind, wie z.B. niedermolekulare Carbonsäuren, Sulfonsäuren oder Fettsäuren. Solche Stoffe finden sich unter Weichmachern, Tackifiern oder Enhancern.

Die Lösung der Aufgabe war möglich, indem statt der zumeist verwendeten wäßrigen Lösungen von Natrium- oder Kaliumhydroxid die entsprechenden alkoholischen, vorzugsweise methanolischen oder ethanolischen, gegebenenfalls einen geringe Wassergehalt enthaltenden Lösungen verwendet wurden. Unter diesen Umständen kann die Reaktion in einem Milieu durchgeführt werden, in dem die oben bezeichneten sauren Polyacrylathaftkleber nicht aus der Lösung ausfallen und auch die Produkte stabil gelöst bleiben.

Möglich ist auch der Einsatz von Alkali-Alkoholaten, z.B. Natriumethanolat oder Kaliummethanolat. Diese alkalischen Reagenzien ermöglichen den völligen Verzicht auf Wasser bei der Neutralisierungsreaktion, und es entsteht auch kein Wasser als Produkt.

Geringer Wassergehalt in Bezug auf die alkoholischen Lösungen heißt, daß der Volumenanteil der Reagenzlösung an Wasser vorzugsweise nicht mehr als 20% und insbesondere nicht mehr als 10% beträgt. Idealerweise werden wasserfreie Lösungen verwendet.

Nach dem Stand der Technik erfolgt der Einsatz alkalisch versalzter oder zumindest im alkalischen Milieu versalzbarer Polyacrylathaftkleber typischerweise im Bereich von wasserdispergierbaren Zubereitungen.

Bei genügend hoher Anzahl von Carboxylgruppen im Polymer läßt sich durch Versalzung die Hydrophilie soweit steigern, daß das Produkt in Wasser dispergiert verarbeitet werden kann.

Bei weiter gesteigertem Gehalt an Carboxylgruppen im Polymer kann sogar eine Löslichkeit im alkalischen, wäßrigen Milieu erreicht werden.

Im Gegensatz dazu enthalten die vorliegend beschriebenen Polyacrylate nur einen geringen Anteil an Carboxylgruppen. Dieser reicht nicht aus, um das neutralisierte Produkt wasserlöslich oder wasserdispergierbar zu machen. Eine solche Dispergierbarkeit oder Löslichkeit in Wasser ist für die Anwendung auf der Haut auch unerwünscht, da ansonsten eine Ablösung schon durch Hautschweiß erfolgen kann.

Auf dem Gebiet technischer und medizinischer Haftkleber sind weiterhin Polyacrylate beschrieben, die ebenfalls einen nur geringen Gehalt von 0,05% bis 8,0% (w/w) carboxylgruppenhaltiger Monomere aufweisen, welche ganz oder teilweise als Alkalisalz vorliegen.

Die Vorteile wurde in hoher Kohäsion, guter Wetter- und Alterungsbeständigkeit gesehen, sowie auch beim Tragen auf perspirierender Haut.

Beobachtungen zu etwaiger Modifikation der Abgabe pharmazeutischer Wirkstoffe aus solchen Matrices liegen aber hier nicht vor.

Ganz ähnlich gelagert ist JP 52-059636 A, die ebenfalls bei einem Polyacrylathaftkleber mit geringem Gehalt an Carboxylgruppen einen Gehalt an alkalisch reagierenden Alkalimetallverbindungen vorsieht. Zusätzlich ist noch eine ionische Quervernetzung vorgesehen.

Beobachtungen zu etwaiger Modifikation der Abgabe pharmazeutischer Wirkstoffe aus solchen Matrices liegen auch hier nicht vor.

Auch in Verbindung mit carboxylgruppenhaltigen pharmazeutischen Wirkstoffen wurde die Technik des Neutralisierens von Polyacrylathaftklebern eingesetzt.

Dort wurde eine Erhöhung der Kohäsion bei Zusatz größerer Mengen von weichmachenden Hilfsstoffen erreicht und insbesondere die Hautverträglichkeit des Pflasters mit dem Wirkstoff Ibuprofen verbessert.

Letzteres wird in Verbindung mit einer Heraufsetzung des pH-Wertes hin zu physiologischen Werten der Hautoberfläche gesehen. Ansonsten könnten Pflaster mit einem carboxylgruppenhaltigen pharmazeutischen Wirkstoff zu stark sauer reagieren und dadurch reizend wirken.

Innerhalb der Gruppe möglicher pharmazeutischer Wirkstoffe wird jedoch ausschließlich auf solche eingegangen, die Carboxylgruppen im Molekül enthalten und somit chemisch sauer reagieren können.

Die Steigerung der Kohäsion, Verbesserung der Klebeigenschaften und der Beständigkeit gegenüber Feuchtigkeit, Erhöhung der Beladbarkeit mit weichmachenden Hilfsstofffen sowie auch die reizmindernde Wirkung in Verbindung mit sauren pharmazeutischen Wirkstoffen waren zusammenfassend als Effekte des Neutralisierens saurer Polyacrylathaftkleber bekannt.

EP 0 446 636 A beschreibt ein transdermales therapeutisches System enthaltend einen basischen Wirkstoff, wie zum Beispiel Diltiazem, und ein haftklebendes Polymer, dessen Gehalt an Carboxylgruppen 10 % oder weniger beträgt, wobei die Carboxylgruppen teilweise oder vollständig mit Alkali neutralisiert sind. Bei dem haftklebenden Polymer handelt es sich jedoch um Copolymere, die zu 90 % aus N-Vinylacetamid bestehen.

WO 99/49852 betrifft ein spezifisches transdermales therpeutisches System zur Verabreichung des Dopamin-D2-Rezeptor-Antagonisten (-)-5,6,7,8-Tetrahydro-6-[propyl[2-(2-thienyl)ethyl]amino]-1-naphthol, bei dem eine Zugabe von NaOH oder KOH dazu dient, das Hydrochloridsalz des (-)-5,6,7,8-Tetrahydro-6-[propyl[2-(2-thienyl)ethyl]amino]-1-naphthols in die freie Base zu überführen, welches die lipophile Barriere des Stratum Corneum wesentlich besser passieren kann. Das Polymerklebersystem dieses spezifischen transdermalen therapeutischen Systems kann auf Polyacrylat- oder Silikonbasis beruhen.

Überraschend wurde nun im Rahmen der vorliegenden Erfindung entdeckt, daß insbesondere die Verwendung basischer pharmazeutischer Wirkstoffe in Kombination mit neutralisierten sauren Polyacrylathaftklebern eine teilweise außerordentliche Steigerung der Abgaberate des pharmazeutischen Wirkstoffs ergibt.

Es ergibt sich weiterhin eine völlig unerwartete Steuerbarkeit der Abgaberate von basischen pharmazeutischen Wirkstoffen über den Neutralisationsgrad des sauren Polyacrylathaftklebers.

Die sich auf dem Gebiet der transdermalen therapeutischen Systeme ergebenden Vorteile einer solchen Kombination übertreffen deutlich die bisher schon erkannten Vorzüge, wie oben geschildert.

Weiterhin wurde völlig unerwartet auch eine Verbesserung - der Abgabe von chemisch neutral reagierenden pharmazeutischen Wirkstoffen gefunden.

Für basische pharmazeutische wirkstoffe ist prinzipiell eine Behinderung der Freisetzung aus einem sauren Polyacrylathaftkleber dadurch zu erwarten, daß eine reversible Bindung an das Polymer durch Säure-Base-Reaktion eintritt. Durch Neutralisieren des Polymers ist eine Lockerung dieser Bindung zwar zu erwarten, jedoch übertrifft das Ausmaß des Effektes und insbesondere seine beobachtete praktisch lineare Steuerbarkeit bei weitem die Erwartungen des Fachmanns.

Bei chemisch neutral reagierenden pharmazeutischen Wirkstoffen ist überhaupt keine Wechselwirkung mit sauren Polyacrylathaftklebern in Art einer Säure-Base-Reaktion zu erwarten. Die überraschend dennoch beobachtete Verbesserung des Freisetzungsverhaltens steht möglicherweise mit Veränderungen der räumlichen Polymerstruktur und der Löslichkeit für darin eingegebettete niedermolekulare Wirkstoffe in Zusammenhang.

Zu den bereits bekannten positiven Effekten des Neutralisierens von sauren Polyacrylathaftklebern, unter denen die Erhöhung der Kohäsion und damit Verbesserung der weichmacherresistenz die größte Rolle spielt, tritt damit ein für basische und neutrale pharmazeutische Wirkstoffe bisher unbekannter positiver Effekt.

Damit ergeben sich als Gegenstand der vorliegenden Erfindung insbesondere neuartige transdermale therapeutische Systeme zur Abgabe basischer oder neutraler pharmazeutischer Wirkstoffe auf der Basis neutralisierter Polyacrylathaftkleber.

Unter basischen pharmazeutischen Wirkstoffen sind solche zu verstehen, die in ihrer Molekülstruktur mindestens eine Gruppe aufweisen, die chemisch als Lewis-Base reagieren kann. Vorzugsweise sind solche Gruppen primäre, sekundäre oder tertiäre aliphatische oder aromatische Amine. Es kann sich aber auch um basisch reagierende Amide oder um Guanidinstrukturen handeln. Beispiele sind Nikotin, Tulobuterol oder Rivastigmin.

Als neutral sind pharmazeutische Wirkstoffe anzusehen, die weder mit Basen noch mit Säuren in eine pharmazeutisch akzeptable Salzform überführt werden können.

Es sind dies beispielsweise steroidale Wirkstoffe wie Testosteron, Norethisteronacetat oder Estradiol sowie organische Ester der Salpetersäure, speziell Nitroglycerin und Isosorbidmononitrat oder -dinitrat.

Wegen der hohen Kohäsion neutralisierter Polyacrylathaftkleber sind solche Formulierungen besonders geeignet für die Einarbeitung flüssiger Wirk- oder Hilfsstoffe, insbesondere Enhancer in größeren Mengen von 10 bis 80% (w/w), vorzugsweise 10 bis 50% (w/w), bezogen auf die Klebermatrix.

Unter der hohen Kohäsion und geringen Fließfähigkeit der neutralisierten Haftkleberfilme kann allerdings die Spontanklebrigkeit auf der Haut nachlassen, so daß solche Filme nicht immer optimale Klebeigenschaften aufweisen.

In solchen Fällen kann es erforderlich sein, hautseitig eine gesonderte Haftkleberschicht vorzusehen, die die Verklebung des Systems mit der Haut verbessert.

Diese Schicht kann prinzipiell aus allen zur medizinischen Anwendung geeigneten Haftklebern aufgebaut sein. Vorzugsweise sollte sie die Freisetzung des pharmazeutischen Wirkstoffes aus der Schicht der neutralisierten Polyacrylatmatrix nicht nennenswert herabsetzen. In diesem Sinne sollte sie in jedem Fall so dünn wie möglich ausgeführt werden. Zum Aufbau dieser hautseitigen Haftkleberschicht geeignete Polymere stammen aus der Gruppe von Polyacrylaten, Silikonkautschuk, Polyisobutylen, Polyisopren sowie Styrol-Isopren-Styrol Blockcopolymeren und Styrol-Butadien-Styrol-Blockcopolymeren.

Die haftklebenden Filme aus neutralisierten Acrylatcopolymeren, insbesondere die mit einem hohen Anteil unveresterter Acryl- oder Methacrylsäure im Polymer (3 bis 10% w/w bezogen auf die mittlere Polymermasse) und einem hohen Grad der Neutralisierung (70 bis 100%), erwiesen sich beim längeren Tragen auf der Haut als zu feuchtigkeitsempfindlich. Dies kann sich in einem vorzeitigen Nachlassen der Klebkraft oder in einer vorzeitigen Ablösung von der Hautoberfläche äußern, verglichen mit der nicht neutralisierten Form derselben Haftkleber.

Dies führt zu einer unerwünschten Anfälligkeit der TTS gegenüber dem Hautschweiß, verbunden mit einer verringerten Eignung für eine Langzeitverwendung für z.B. mindestens 16 h. Insbesondere bei TTS für Langzeitanwendung (für 24 h oder bei sog. 2- oder 3-Tage-Pflastern) kann dies nachteilig sein.

Der Problematik der Feuchtigskeitsempfindlichkeit konnte jedoch durch Beimischung von stark wasserbindenden Zusätzen begegnet werden. Als geeignete Zusätze, die in hohem Maße Wasser zu adsorbieren vermögen, ohne sich darin zu lösen, kommen pharmazeutisch akzeptable Quellstoffe, vorzugsweise Natrium- oder Calciumsalze der quervernetzten Carboxymethylcellulose (Croscarmellose), Natrium- oder Calciumsalze von quervernetzter Polyacrylsäure oder quervernetztes Polyvinylpyrrolidon (Crospovidon) in Frage.

Weitere Beispiele sind Produkte wie Galaktomannane, Celluloseprodukte, Tragant, Polyglycoside, feinpulverisierte Polyamide, wasserlösliches Polyacrylamid, Carboxyvinylpolymerisate, agar-ähnliche Algenprodukte, Mischpolymerisate aus Methylvinylether und Maleinsäureanhydrid, Guar-Gummi, Hydroxypropylguar-Gummi oder Guar-Mehl, Gummi arabicum, Dextrin und Dextran, mikrobiologisch gewonnenes Polysaccharid-Gummi wie das Polysaccharid B 1459 oder die gut wasserlösliche Type Keltrol bzw. synthetisch gewonnene Polysaccharide wie das Produkt Ficoll, Methylglucosederivate, Hydroxymethylpropylcellulose, Polygalakturonsäurederivate wie Pectin oder das amidierte Produkt Pectinamid.

Hiervon sind Galaktomannane und mikrokristalline Cellulose besonders bevorzugt.

Die Quellungszahl solcher Stoffe, bestimmt nach dem Europäischen Arzneibuch 1997, sollte mindestens den Wert 2 ergeben, vorzugsweise aber größer als 4 sein. Die Teilchengröße sollte 1 bis 50 µm, vorzugsweise aber 5 bis 25 µm betragen. Dadurch ist gewährleistet, daß flüssige Kleberlösungen, die ein solches Mittel enthalten, auch in geringen Schichtdicken von zumeist 100 bis 1000 µm einwandfrei beschichtet werden können.

Die zugesetzte Menge eines wasserbindenden Mittels sollte so gering wie nötig gehalten werden, da das von diesem Mittel im TTS beanspruchte Volumen in der Regel für die Beladung mit Wirkstoff verloren geht. Mengen von 0,1 bis 5 Gew.-% (bezogen auf das Gesamtgewicht des wirkstoffhaltigen, haftklebenden Polymerfilms) eines solchen wasserbindenden Zusatzstoffes reichen im allgemeinen für diesen Zweck aus.

Die Erfindung wird im folgenden anhand von Ausführungsbeispielen erläutert.

Untersuchungen zur Thermodynamik am Modell der Permeation von Ethylvinylacetat(EVA)copolymerfolie

Für drei basische pharmazeutische Wirkstoffe wurde das Freisetzungsverhalten aus einem sauren Polyacrylathaftkleberfilm in Abhängigkeit von Neutralisation des Klebers und des dazu verwendeten Reagenzes untersucht.

Alle versuche auf EVA-Membran wurden mit n=3 Mustern durchgeführt.

Dazu wurden die wirkstoffhaltigen Haftkleberfilme auf eine EVA-Folie als Trägermembran geklebt und die über die Zeit durch diese Membran hindurch an ein auf der Gegenseite liegendes, wäßriges, auf pH 5,5 gepuffertes Medium abgegebene Wirkstoffmenge wurde durch geeignete HPLC-Methoden quantifiziert.

Gegenüber biologischen Membranen wie Haut besitzt die EVA-Folie den Vorteil hoher Standardisierbarkeit, so daß äußerst aussagekräftige vergleichsmessungen möglich werden. Bei der Permeationsapparatur handelte es sich um eine modifizierte Permeationszelle nach Franz, wie Sie auf dem Gebiet der Entwicklung von TTS allgemein bekannt ist.

Die Verwendung von EVA-Folie als Membran erlaubt in diesem versuchsdesign die isolierte Betrachtung der thermodynamischen Aktivität eines Wirkstoffes in der haftklebenden Matrix.

Da der Wirkstoff in den Ausführungsbeispielen die einzige niedermolekulare und wanderungsfähige Komponente bildet, wird allein sein Bestreben erfaßt, aus der Polymermatrix des Haftklebers auszutreten und durch die EVA-Membran hindurch in das Aufnahmemedium zu wandern.

Unabhängig von den Durchlaßeigenschaften der menschlichen Haut für eine solchen Wirkstoff ist das hier gemessene Abgabebestreben des Systems gegenüber dem Wirkstoff die wesentlichste Treibkraft für die transdermale Therapie mittels eines solchen Systems.

Die getesteten TTS bestanden alle aus einer wirkstoffhaltigen Haftkleberschicht mit einem Flächengewicht von 80 g/m². Der Wirkstoffgehalt betrug bei Tulobuterol, Rivastigmin und Xanomelin 5% (w/w), bei Testosteron betrug er 2,5% (w/w). Zur Herstellung wurde die kommerziell erworbene Haftkleberlösung Durotak^{®} 387-2051 (Fa. National Starch) gegebenenfalls mit einer 10%igen (w/w) methanolischen Lösung des Alkalihydroxides in einer Menge vermischt, die einem Neutralisationsgrad für das Polyacrylat von 100% entspricht.

Erst danach wurde der Wirkstoff zugegeben und in der Masse aufgelöst.

Diese Lösung wurde auf eine silikonisierte Trägerfolie aus Polyethylenterephthalat (PET) beschichtet und in einem Abluftofen 10 Minuten lang bei 80°C zu einem Film getrocknet. Der trockene Film wurde mit einer 15 µm dicken PET-Folie abgedeckt.

Geeignete Stanzlinge dieses Laminates wurden entnommen und die Trägerfolie entfernt, bevor der wirkstoffhaltige Film auf die EVA-Trägerfolie geklebt wurde.

**Tabelle 1: Formulierungen für EVA-Permeationen**

| Rez.-Nr. | Wirkstoff | Neutralisier-Reagenz | Neutralisationsgrad [%] |
|---|---|---|---|
| 1 | Tulobuterol | --- | 0 |
| 2 | Tulobuterol | KOH | 100 |
| 3 | Tulobuterol | NaOH | 100 |
| 4 | Rivastigmin | --- | 0 |
| 5 | Rivastigmin | KOH | 100 |
| 6 | Rivastigmin | NaOH | 100 |
| 7 | Xanomelin | --- | 0 |
| 8 | Xanomelin | KOH | 100 |
| 9 | Xanomelin | NaOH | 100 |
| 10 | Rivastigmin | KOH | 20 |
| 11 | Rivastigmin | KOH | 40 |
| 12 | Rivastigmin | KOH | 60 |
| 13 | Rivastigmin | KOH | 80 |
| 14 | Testosteron | KOH | 0 |
| 15 | Testosteron | KOH | 50 |
| 16 | Testosteron | KOH | 100 |

| | | | |
|---|---|---|---|
| NaOH=Natriumhydroxid; KOH=Kaliumhydroxid | | | |

### Beispiel Nr. 17 mit dem Wirkstoff Nikotin:

Dieses Beispiel zeigt ein zweischichtiges Matrix TTS mit dem basischen Wirkstoff Nikotin. In der neutralisierten Acrylatcopolymer-Haftkleberschicht ist ein wasserbindender Zusatzstoff (sog. Hydroadsorbens, im speziellen Fall: Croscarmellose-Natrium, d.h. das Natriumsalz von quervernetzter Carboxymethylcellulose) enthalten. Das fertige TTS klebte über einen Tragezeitraum von 24 Stunden einwandfrei ohne Ablösungen von der Haut.

Die Polymerlösung für die erste Matrixschicht weist die Zusammensetzung gemäß Tabelle 2 auf. Diese Lösung wird durch ein Auftragswerk flächig auf eine Folie aus Polyethylenterephthalat (19 µm PET) mit einem Flächengewicht von 54 g/m² beschichtet. Dieses Beschichtungsprodukt wird sofort anschließend mit einer Haftkleberschicht der Zusammensetzung gemäß Tabelle 3 und einer Schichtdicke von 144 g/m² zusammenkaschiert.

Dieses Kaschierprodukt wird 10 Minuten auf 60°C erwärmt, bevor die Aufwickelung zu einem Rollenprodukt erfolgt. Das Rollenprodukt wird sofort oder auch nach Zwischenlagerung in üblicher weise durch Längsschneiden und Stanzen zu TTS weiterverarbeitet.

**Tabelle 2:**

| Bezeichnung | Menge [%] | Funktion |
|---|---|---|
| Nikotin | 32,41 | Basischer Wirkstoff |
| Eudragit® EPO | 27,00 | verdickendes Polymer |
| Miglyol® 812 | 40,23 | Kosolvens |
| Vitamin E | 0,36 | Antioxidans |

**Tabelle 3:**

| Bezeichnung | Menge [%] | Funktion |
|---|---|---|
| Durotak 387-2051 | 95,18 | Acrylatcopolymer-Haftkleber |
| KOH | 0,77 | Neutralisationsreagenz |
| Aluminiumacetylacetonat | 0,05 | Vernetzungsreagenz für das Acrylatcopolymer Durotak |
| Croscarmellose-Natrium | 4,00 | Hydroadsorbens |

Die Herstellung der Kleberschicht gemäß Tabelle 3 erfolgtin einem konventionellen, lösemittelhaltigen Beschichtungsverfahren mit anschließender Trocknung. Prozeßlösemittel sind Ethylacetat, Methanol und Acetylaceton. Die angegebenen Mengenanteile beziehen sich auf den Gehalt in der getrockneten Schicht.

Durch Diffusion wandern die flüssigen Bestandteile der ersten Matrixschicht (wie z.B. der Wirkstoff und das Cosolvens) in die zunächst wirkstofffreie zweite Matrixschicht. Nach wenigen Tagen ist dieser Prozess jedoch abgeschlossen und das fertige TTS einsatzbereit.

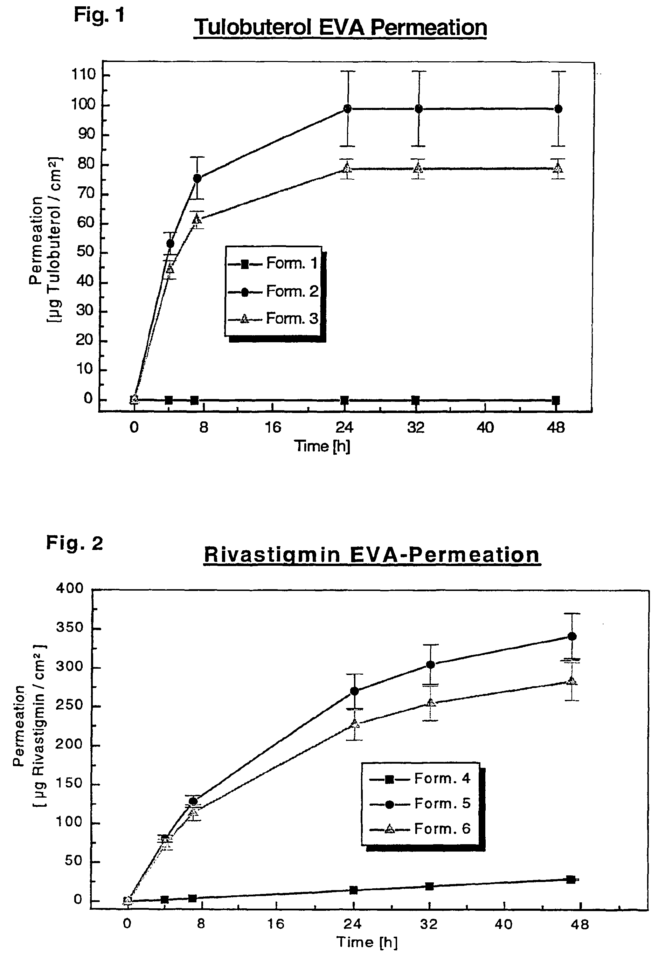

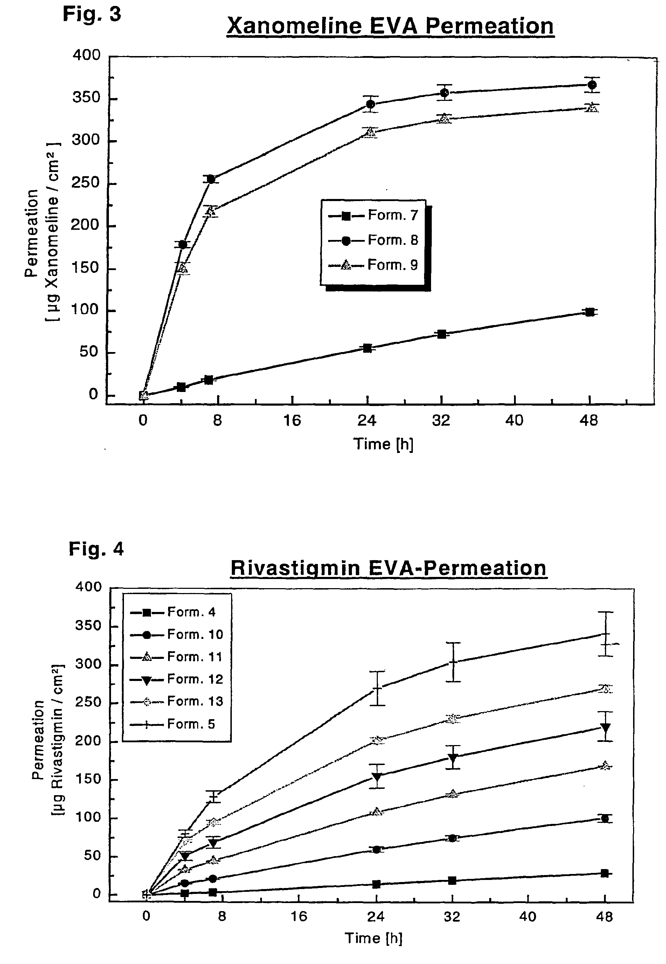

Die Abbildungen 1 bis 3 zeigen für die drei untersuchten basischen Wirkstoffe der Beispiele 1 bis 16 eine extreme Zunahme der Freisetzung aus der haftklebenden Matrix, wenn das Polyacrylat neutralisiert vorliegt.

Weiterhin ist ein eindeutiger Vorteil bei der Verwendung von Kaliumhydroxid gegenüber Natriumhydroxid zu erkennen.

Abbildung 4 zeigt für den Wirkstoff Rivastigmin beispielhaft die starke Abhängigkeit des Effektes von dem Neutralisationsgrad.

Abbildung 5 zeigt dieselben Daten wie Abbildung 4, jedoch unter direkter Auftragung der permeierten Wirkstoffmengen gegen den Neutralisationsgrad (Anm.: Neutralisationsgrade von 0 bis 100% entsprechen den Formulierungen 4,10,11,12,13,5 in dieser Reihenfolge).

Hierin kann sehr überraschend erkannt werden, daß praktisch über den gesamten Bereich von 0 bis 100% Neutralisationsgrad eine lineare Abhängigkeit besteht. Diese wird durch gestrichelt eingezeichnete lineare Regressionen verdeutlicht.

Die pro Zeit freizusetzende Menge des Wirkstoff kann folglich präzise über den Neutralisationsgrad gesteuert werden.

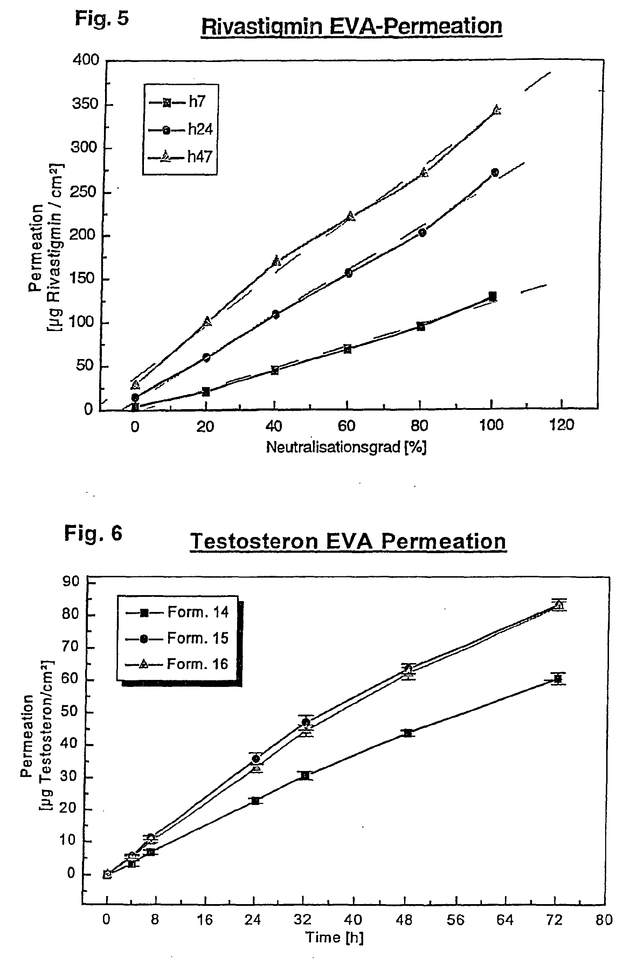

Abbildung 6 zeigt die Auswirkung der Neutralisierung des Haftklebers auf die Abgabe eines neutralen Wirkstoffs. Auch hier ist eine deutliche Erhöhung der Abgaberaten durch Neutralisierung sichtbar. Im Gegensatz zu den untersuchten basischen Wirkstoffen ist offenbar keine lineare Abhängigkeit der Abgabeleistung vom Neutralisationsgrad gegeben.

## Patentansprüche

1. Transdermales therapeutisches System in der Ausführungsform eines Matrix- oder Reservoirsystems, umfassend
einen Gehalt an mindestens einem basisch oder neutral reagierenden pharmazeutischen Wirkstoff und
einen Gehalt an einem ganz oder teilweise neutralisierten sauren Polyacrylathaftkleber, welcher als Teil der Kette Acrylsäure- oder Methacrylsäureeinheiten aufweist, wobei
- der Gehalt an Carboxylgruppen, bezogen auf die mittlere Polymermasse, 0,5 bis 10,0 % (w/w) beträgt und die Carboxylgruppen stöchiometrisch zu 5 bis 100 % (w/w), vorzugsweise 10 bis 50 %, in Form von Alkalisalzen oder Erdalkalisalzen vorliegen,
- es sich bezogen auf die mittlere Polymermasse bei mindestens 50 % (w/w) davon um Monomeren aus der Gruppe von Acryl- oder Methacrylsäure oder deren Esterderivaten handelt, und
- es sich bezogen auf die mittlere Polymermasse bei mindestens 0,5 % (w/w), höchstens aber 10 % (w/w), davon um unveresterte Acryl- oder Methacrylsäuren handelt,
**dadurch gekennzeichnet, dass**
die im sauren Polyacrylathaftkleber enthaltenen Carboxylgruppen ganz oder teilweise neutralisiert wurden, indem sie in die Alkali- oder Erdalkalisalze umgewandelt wurden, wobei zur Umwandlung alkalische Verbindungen der Alkalimetalle oder Erdalkalimetalle dienten, und alkoholische, gegebenenfalls einen geringen Wassergehalt von nicht mehr als 20 Volumenprozent enthaltende Lösungen oder Alkali-Alkoholate verwendet wurden.

2. Transdermales therapeutisches System gemäß Anspruch 1, **gekennzeichnet dadurch, daß** es sich um ein Matrixsystem handelt, bestehend aus einer nicht-klebenden Rückschicht, ein bis drei, vorzugsweise einer oder zwei haftklebenden Schicht(en) und einer dehäsiv ausgerüsteten, wiederablösbaren Schutzschicht.

3. Transdermales therapeutisches System nach einem der vorangehenden Ansprüche, **gekennzeichnet dadurch, daß** es sich bei dem Alkalisalz um das Natrium- oder Kaliumsalz handelt.

4. Transdermales therapeutisches System nach einem der vorangehenden Ansprüche, **gekennzeichnet dadurch, daß** es sich bei dem Erdalkalisalz um das Magnesium- oder Calciumsalz handelt.

5. Transdermales therapeutisches System nach einem der vorangehenden Ansprüche, **gekennzeichnet dadurch, daß** der pharmazeutische Wirkstoff bei 20°C als Flüssigkeit vorliegt.

6. Transdermales therapeutisches System nach Anspruch 5, **gekennzeichnet dadurch, daß** der pharmazeutische Wirkstoff in einer Menge von 2 bis 50% (m/m), vorzugsweise 5 bis 25% (m/m), bezogen auf die wirkstoffhaltige Matrix, enthalten ist.

7. Transdermales therapeutisches System nach einem der vorangehenden Ansprüche, **gekennzeichnet dadurch, daß** ein basischer pharmazeutischer Wirkstoff in Form eines pharmazeutisch akzeptablen Salzes, vorzugsweise als Acetat, Citrat, Fumarat, Hydrochlorid, Lactat, Maleat, Mesylat, Sulfat oder Tartrat enthalten ist.

8. Transdermales therapeutisches System nach einem der vorangehenden Ansprüche, **gekennzeichnet dadurch, daß** das bezeichnete carboxylgruppenhaltige Polymer mit Aluminiumionen in einer Konzentration von 0,005 bis 0,5% (m/m), vorzugsweise 0,01 bis 0,1% (m/m), berechnet als Aluminium, bezogen auf die Polymermasse, quervernetzt vorliegt.

9. Transdermales therapeutisches System nach Anspruch 8, **gekennzeichnet dadurch, daß** das eingesetzte Quervernetzungsreagenz Aluminiumacetylacetonat ist.

10. Transdermales therapeutisches System nach einem der vorangehenden Ansprüche, **gekennzeichnet dadurch, daß** die wirkstoffhaltige Matrix einen Gehalt an mindestens einem Enhancer aus der Gruppe von geradkettigen oder verzweigtkettigen Fettalkoholen der allgemeinen Formel CₓH_{y}CH₂OH mit X=9 bis 17 und Y=19 bis 33, vorzugsweise Decanol, Dodecanol, 2-Hexyldecanol, 2-Octyldodecanol oder Oleylalkohol, aufweist.

11. Transdermales therapeutisches System nach einem der vorangehenden Ansprüche, **gekennzeichnet dadurch, daß** die wirkstoffhaltige Matrix einen Gehalt an mindestens einem Enhancer aus der Gruppe von gesättigten oder ungesättigten Fettsäuren der allgemeinen Formel CₓC_{y}OOH mit X=9 bis 17 und Y=19 bis 33, vorzugsweise Undecylensäure, Laurinsäure, Myristinsäure oder Ölsäure aufweist.

12. Transdermales therapeutisches System nach einem der vorangehenden Ansprüche, **gekennzeichnet dadurch, daß** die wirkstoffhaltige Matrix einen Gehalt an mindestens einem Enhancer aus der Gruppe von Sorbitanfettsäureestern oder deren durch Ethoxylierung erhältlichen Derivaten aufweist, vorzugsweise Sorbitanmonolaurat und Sorbitanmonooleat, die jeweils mit 5 bis 20 Molekülen Ethylenoxid pro Sorbitanestermolekül verethert vorliegen können.

13. Transdermales therapeutisches System nach einem der vorangehenden Ansprüche, **gekennzeichnet dadurch, daß** die wirkstoffhaltige Matrix einen Gehalt an mindestens einem Enhancer aus der Gruppe von Fettalkoholethoxylaten aufweist, vorzugsweise die Umsetzungsprodukte von Dodecanol oder Oleylalkohol mit 1-5 Einheiten Ethylenoxid.

14. Transdermales therapeutisches System nach einem der vorangehenden Ansprüche, **gekennzeichnet dadurch, daß** die wirkstoffhaltige Matrix einen Gehalt an mindestens einem Enhancer aus der Gruppe von Estern von Fettsäuren mit Methanol, Ethanol oder Isopropanol aufweist, vorzugsweise Methyllaurat, Ethyloleat, Isopropylmyristat oder Isopropylpalmitat.

15. Transdermales therapeutisches System nach einem der vorangehenden Ansprüche, **gekennzeichnet dadurch, daß** die wirkstoffhaltige Matrix einen Gehalt an mindestens-einem Enhancer aus der Gruppe von Estern von Fettalkoholen mit Essigsäure oder Milchsäure aufweist, vorzugsweise Lauryllactat oder Oleylacetat.

16. Transdermales therapeutisches System nach einem der vorangehenden Ansprüche, **gekennzeichnet dadurch, daß** die wirkstoffhaltige Matrix einen Gehalt an mindestens einem mit Wasser mischbaren Enhancer aus der Gruppe von mehrwertigen aliphatischen Alkoholen oder Polyethylenglykolen aufweist, vorzugsweise 1,2-Propylenglykol, Glycerin, 1,3-Butandiol, Dipropylenglykol sowie Polyethylenglykole mit mittleren Molekulargewichten von 200 bis 600 Da.

17. Transdermales therapeutisches System nach Anspruch 16 **gekennzeichnet dadurch, daß** der genannte Enhancer ganz oder teilweise in Art einer Emulsion dispergiert in der Matrix vorliegt, wobei der Enhancer vorzugsweise Glycerin ist.

18. Transdermales therapeutisches System nach einem der vorangehenden Ansprüche, **gekennzeichnet dadurch, daß** die wirkstoffhaltige Matrix einen Gehalt an mindestens einem Weichmacher aus der Gruppe der Citronensäureester oder der gesättigten Triglyceride aufweist, vorzugsweise Triethylcitrat, Acetyltributylcitrat, Triacetin oder mittelkettige Triglyceride mit Fettsäurekettenlängen von 8 bis 12 Kohlenstoffatomen.

19. Transdermales therapeutisches System nach einem oder mehreren der Ansprüche 10 bis 18, **gekennzeichnet dadurch, daß** der Enhancer oder ein Gemisch der genannten Enhancer oder ein Gemisch der genannten mit nicht-genannten Enhancern 10 bis 80%, vorzugsweise 10-50% (m/m) der wirkstoffhaltigen Matrix ausmacht.

20. Transdermales therapeutisches System nach einem der vorangehenden Ansprüche, **gekennzeichnet dadurch, daß** im System ein Wasser adsorbierendes oder absorbierendes festes Mittel im trockenen Zustand enthalten ist, vorzugsweise Natrium- oder Calciumsalze von Carboxymethylcellulose, quervernetztes Polyvinylpyrrolidon, Natrium- oder Calciumsalze von quervernetzter Polyacrylsäure bzw. Polymethacrylsäure, sowie Natrium- oder Calciumsalze von Carboxymethylstärke.

21. Transdermales therapeutisches System nach einem der vorangehenden Ansprüche, **gekennzeichnet dadurch, daß** eine separate hautseitig haftklebende Schicht vorgesehen ist, bestehend aus einem carboxylgruppenhaltigen Acrylathaftkleber, dessen Carboxylgruppen jedoch nicht als Salz vorliegen und der vorzugsweise durch Aluminiumionen quervernetzt ist.

22. Transdermales therapeutisches System nach einem der vorangehenden Ansprüche, **gekennzeichnet dadurch, daß** eine separate hautseitig haftklebende Schicht vorgesehen ist, bestehend aus einem carboxylgruppenfreien und hydroxylgruppenhaltigen Acrylathaftkleber, der vorzugsweise durch Aluminiumionen oder Titanionen quervernetzt ist.

23. Transdermales therapeutisches System nach einem der vorangehenden Ansprüche, **gekennzeichnet dadurch, daß** eine separate hautseitig haftklebende Schicht aus einem Haftkleber auf Silikonbasis enthalten ist.

24. Transdermales therapeutisches System nach einem der vorangehenden Ansprüche, **gekennzeichnet dadurch, daß** eine separate hautseitig haftklebende Schicht vorgesehen ist, bestehend aus einem Haftkleber auf Basis einer Mischung von Polyisobutylenen mit mindestens 2 verschiedenen mittleren Molekulargewichten.

25. Transdermales therapeutisches System nach Anspruch 21 bis 24, **gekennzeichnet dadurch, daß** die hautseitig haftklebende Schicht ein Flächengewicht von 10 bis 100 g/m², vorzugsweise 20 bis 50 g/m² aufweist.

26. Transdermales therapeutisches System nach einem der vorangehenden Ansprüche, **gekennzeichnet dadurch, daß** der pharmazeutische Wirkstoff Nikotin, Xanomeline oder Rivastigmin ist.

27. Transdermales therapeutisches System nach einem der vorangehenden Ansprüche, **gekennzeichnet dadurch, daß** der pharmazeutische Wirkstoff ein Steroidhormon, vorzugsweise Estradiol, Levonorgestrel, Norethisteronacetat, Gestoden oder Testosteron ist.

28. Transdermales therapeutisches System nach einem der vorangehenden Ansprüche, **gekennzeichnet dadurch, daß** der pharmazeutische Wirkstoff ein organischer Ester der Salpetersäure, vorzugsweise Nitroglycerin, Isosorbidmononitrat oder Isosorbiddinitrat ist.

## Claims

1. Transdermal therapeutic system embodied as a matrix or reservoir system, comprising
a content of at least one basic- or neutral-reacting pharmaceutical active agent, and
a content of a completely or partially neutralised, acid polyacrylate pressure-sensitive adhesive which possesses, as part of its chain, acrylic acid or methacrylic acid units,
- the content of the carboxyl groups, relative to the mean polymer mass, being 0.5 to 10.0% (w/w) and the carboxyl groups being present stoichiometrically at 5 to 100% (w/w), preferably 10 to 50%, in the form of alkali salts or alkaline earth salts,
- at least 50% (w/w) thereof, relative to the mean polymer mass, being monomers from the group of acrylic or methacrylic acid or ester derivatives thereof, and
- 0.5% (w/w), but at maximum 10% (w/w), thereof, relative to the mean polymer mass, being unesterified acrylic or methacrylic acids,
**characterised in that**
the carboxyl groups contained in the acid polyacrylate pressure-sensitive adhesive have been partially or completely neutralized by converting them to the alkali salts or alkaline earth salts, with alkaline compounds of alkali metals or alkaline earth metals, as well as alcoholic solvents, possibly containing a low content of water not exceeding 20% by volume, or alkali alcoholates being used for the conversion.

2. Transdermal therapeutic system according to Claim 1, **characterized in that** it is a matrix system consisting of a non-adhesive backing layer, one to three, preferably one or two, pressure-sensitive adhesive layer(s), and a removable protective layer rendered dehesive.

3. Transdermal therapeutic system according to any one of the preceding claims, **characterized in that** the alkali salt is the sodium or potassium salt.

4. Transdermal therapeutic system according to any one of the preceding claims, **characterized in that** the alkaline earth metal is the magnesium or calcium salt.

5. Transdermal therapeutic system according to any one of the preceding claims, **characterized in that** the pharmaceutical active substance is present, at 20°C, as a liquid.

6. Transdermal therapeutic active system according to Claim 5, **characterized in that** the pharmaceutical active agent is present in an amount from 2 to 50% (m/m), preferably 5 to 25% (m/m), relative to the active substance-containing matrix.

7. Transdermal therapeutic system according to any one of the preceding claims, **characterized in that** it contains a basic pharmaceutical active substance in the form of a pharmaceutically acceptable salt, preferably as acetate, citrate, fumarate, hydrochloride, lactate, maleate, mesylate, sulfate or tartrate.

8. Transdermal therapeutic system according to any one of the preceding claims, **characterized in that** the carboxyl group-containing polymer mentioned is present crosslinked with aluminium ions in a concentration of 0.005 to 0.5% (m/m), preferably 0.01 to 0.1% (m/m), calculated as aluminium, relative to the polymer mass.

9. Transdermal therapeutic system according to Claim 8, **characterized in that** the crosslinking reagent utilised is aluminium acetyl acetonate.

10. Transdermal therapeutic system according to any one of the preceding claims, **characterized in that** the active substance-containing matrix has a content of at least one enhancer from the group of straight-chain or branched-chain fatty alcohols of the general formula C_{X}H_{Y}CH₂OH where X = 9 to 17 and Y = 19 to 33, preferably decanol, dodecanol, 2-hexyl decanol, 2-octyl dodecanol or oleyl alcohol.

11. Transdermal therapeutic system according to any one of the preceding claims, **characterized in that** the active substance-containing matrix has a content of at least one enhancer from the group of saturated or unsaturated fatty acids of the general formula C_{X}H_{Y}COOH where X = 9 to 17 and Y = 19 to 33, preferably undecylenic acid, lauric acid, myristic acid or oleic acid.

12. Transdermal therapeutic system according to any one of the preceding claims, **characterized in that** the active substance-containing matrix has a content of at least one enhancer from the group of sorbitan fatty acid esters or of their derivatives obtained by ethoxylation, preferably sorbitan monolaurate and sorbitan mono-oleate, which may each be present etherified with 5 to 20 molecules of ethylene oxide per sorbitan ester molecule.

13. Transdermal therapeutic system according to any one of the preceding claims, **characterized in that** the active substance-containing matrix has a content of at least one enhancer from the group of fatty alcohol ethoxylates, preferably the reaction products of dodecanol or oleyl alcohol with 1 to 5 units of ethylene oxide.

14. Transdermal therapeutic system according to any one of the preceding claims, **characterized in that** the active substance-containing matrix has a content of at least one enhancer from the group of esters of fatty acids with methanol, ethanol or isopropanol, preferably methyl laurate, ethyl oleate, isopropyl myristate or isopropyl palmitate.

15. Transdermal therapeutic system according to any one of the preceding claims, **characterized in that** the active substance-containing matrix has a content of at least one enhancer from the group of esters of fatty alcohols with acetic acid or lactic acid, preferably lauryl lactate or oleyl acetate.

16. Transdermal therapeutic system according to any one of the preceding claims, **characterized in that** the active substance-containing matrix has a content of at least one water-miscible enhancer from the group of polyvalent aliphatic alcohols or polyethylene glycols, preferably 1,2-propylene glycol, glycerine, 1,3-butanediol, dipropylene glycol as well as polyethylene glykols with mean molecular weights of 200 to 600 Da.

17. Transdermal therapeutic system according to Claim 16, **characterized in that** the so-called enhancer is present in the matrix dispersed partially or completely, as in the case of an emulsion, the enhancer preferably being glycerine.

18. Transdermal therapeutic system according to any one of the preceding claims, **characterized in that** the active substance-containing matrix has a content of at least one plasticizer from the group of the citric acid esters or the saturated triglycerides, preferably triethyl citrate, acetyl tributyl citrate, triacetin or medium-chain triglycerides with fatty acid chains having a length of 8 to 12 carbon atoms.

19. Transdermal therapeutic system according to one or more of claims 10 to 18, **characterized in that** the enhancer, or a mixture of the enhancers mentioned, or a mixture of the enhancers mentioned with enhancers not mentioned, accounts for 10 to 80%, preferably 10 to 50% (m/m) of the active substance-containing matrix.

20. Transdermal therapeutic system according to any one of the preceding claims, **characterized in that** the system contains dispersed therein a water-adsorbing or water-absorbing solid agent in dry condition, preferably sodium or calcium salts of carboxymethyl cellulose, crosslinked polyvinyl pyrrolidone, sodium or calcium salts of crosslinked polyacrylic acid or polymethacrylic acid, as well as sodium or calcium salts of carboxymethyl starch.

21. Transdermal therapeutic system according to any one of the preceding claims, **characterized in that** there is provided a separate layer which is pressure-sensitive adhesive on the skin-facing side and consisting of a carboxyl group-containing pressure-sensitive acrylate adhesive the carboxyl groups of which are, however, not present as a salt and which is preferably crosslinked by aluminium ions.

22. Transdermal therapeutic system according to any one of the preceding claims, **characterized in that** there is provided a separate layer which is pressure-sensitive adhesive on the skin-facing side and consisting of a carboxyl group-free and hydroxyl group-containing pressure-sensitive acrylate adhesive which is preferably crosslinked by aluminium ions or titanium ions.

23. Transdermal therapeutic system according to any one of the preceding claims, **characterized in that** it contains a separate layer which is pressure-sensitive adhesive on the skin-facing side and consists of a silicone-based pressure-sensitive adhesive.

24. Transdermal therapeutic system according to any one of the preceding claims, **characterized in that** there is provided a separate layer which is pressure-sensitive adhesive on the skin-facing side and consisting of a pressure-sensitive adhesive based on a mixture of polyisobutylenes having at least two different medium molecular weights.

25. Transdermal therapeutic system according to Claims 21 to 24, **characterized in that** the said layer, which is pressure-sensitive adhesive on the skin-facing side, has a weight per unit area of 10 to 100 g/m²*,* preferably 20 to 50 g/m².

26. Transdermal therapeutic system according to any one of the preceding claims, **characterized in that** the pharmaceutical active substance is nicotine, xanomeline or rivastigmin.

27. Transdermal therapeutic system according to any one of the preceding claims, **characterized in that** the pharmaceutical active substance is a steroid hormone, preferably estradiol, levonorgestrel, norethisterone acetate, gestodene or testosterone.

28. Transdermal therapeutic system according to any one of the preceding claims, **characterized in that** the pharmaceutical active agent is an organic ester of nitric acid, preferably nitroglycerin, isosorbide mononitrate or isosorbide dinitrate.

## Revendications

1. Système thérapeutique transdermique ayant la forme d'un système de matrice ou de réservoir, contenant
au moins un principe actif pharmaceutique à réaction basique ou neutre et
une colle de fixation à base de polyacrylate acide totalement ou partiellement neutralisé, qui présente, en tant que motifs récurrents de la chaîne, des unités acide acrylique ou méthacrylique,
- la teneur en groupes carboxyle étant, par rapport à la masse moyenne du polymère, de 0,5 à 10,0 % (p/p) et les groupes carboxyle se trouvant dans une proportion stoechiométrique 5 à 100 % (p/p), de préférence de 10 à 50 %, sous la forme de sels de métaux alcalins ou alcalino-terreux,
- par rapport à la masse moyenne du polymère, au moins 50 % (p/p) de ces derniers étant des monomères appartenant au groupe des acides acrylique ou méthacrylique ou de leurs esters, et,
- par rapport à la masse moyenne du polymère, au moins 0,5 % (p/p), mais tout au plus 10 % (p/p) étant sous forme d'acide acrylique ou méthacrylique non estérifié,
**caractérisé en ce que** les groupes carboxyle qui se trouvent dans la colle de fixation à base de polyacrylate acide ont été partiellement ou totalement neutralisés par transformation en leur sel de métal alcalin ou alcalino-terreux, la transformation étant réalisée à l'aide de composés alcalins de métaux alcalins ou alcalino-terreux, avec la mise en oeuvre de solutions alcooliques contenant éventuellement une faible proportion d'eau qui ne dépasse pas 20 % en volume, ou des alcoolates de métaux alcalins.

2. Système thérapeutique transdermique selon la revendication 1, **caractérisé en ce qu'**il s'agit d'un système de matrice, constitué par une couche de base non adhésive, une à trois, de préférence une ou deux couches de fixation adhésives et une couche de protection détachable, pelliculable.

3. Système thérapeutique transdermique selon l'une quelconque des revendications précédentes, **caractérisé en ce que** le sel de métal alcalin est un sel de sodium ou de potassium.

4. Système thérapeutique transdermique selon l'une quelconque des revendications précédentes, **caractérisé en ce que** le sel de métal alcalino-terreux est un sel de magnésium ou de calcium.

5. Système thérapeutique transdermique selon l'une quelconque des revendications précédentes, **caractérisé en ce que** le principe actif pharmaceutique est liquide à 20°C.

6. Système thérapeutique transdermique selon la revendication 5, **caractérisé en ce que** le principe actif pharmaceutique est présent dans une proportion de 2 à 50 % (m/m), de préférence de 5 à 25 % (m/m), par rapport à la matrice contenant le principe actif.

7. Système thérapeutique transdermique selon l'une quelconque des revendications précédentes, **caractérisé en ce qu'**il contient un principe actif pharmaceutique basique sous la forme d'un sel pharmaceutiquement acceptable, de préférence sous forme d'acétate, de citrate, de fumarate, de chlorhydrate, de lactate, de maléate, de mésylate, de sulfate ou de tartrate.

8. Système thérapeutique transdermique selon l'une quelconque des revendications précédentes, **caractérisé en ce que** ledit polymère contenant des groupes carboxyle se trouve sous forme réticulée par des ions aluminium à une concentration de 0,005 à 0,5% (m/m), de préférence de 0,01 à 0,1% (m/m), calculé en tant qu'aluminium, par rapport à la masse du polymère.

9. Système thérapeutique transdermique selon la revendication 8, **caractérisé en ce que** le réactif de réticulation mis en oeuvre est de l'acétylacétonate d'aluminium.

10. Système thérapeutique transdermique selon l'une quelconque des revendications précédentes, **caractérisé en ce que** la matrice contenant le principe actif présente une teneur en au moins un stimulateur (« Enhancer ») appartenant au groupe des alcools gras à chaîne linéaire ou ramifiée, de formule générale CₓH_{y}CH₂OH où X=9 à 17 et Y=19 à 33, de préférence le décanol, le dodécanol, le 2-hexyldécanol, le 2-octyldodécanol ou l'alcool oléylique.

11. Système thérapeutique transdermique selon l'une quelconque des revendications précédentes, **caractérisé en ce que** la matrice contenant le principe actif présente une teneur en au moins un stimulateur appartenant au groupe des acides gras saturés ou insaturés de formule générale CₓH_{y}COOH où X=9 à 17 et Y=19 à 33, de préférence l'acide undécylénique, laurique, myristique ou oléique.

12. Système thérapeutique transdermique selon l'une quelconque des revendications précédentes, **caractérisé en ce que** la matrice contenant le principe actif présente une teneur en au moins un stimulateur appartenant au groupe des esters d'acide gras et de sorbitan ou de leurs dérivés éthoxylés, de préférence le monolaurate de sorbitan et le monooléate de sorbitan, qui peuvent être chacun étherifiés avec 5 à 20 molécules d'oxyde d'éthylène par molécule d'ester de sorbitan.

13. Système thérapeutique transdermique selon l'une quelconque des revendications précédentes, **caractérisé en ce que** la matrice contenant le principe actif présente une teneur en au moins un stimulateur appartenant au groupe des éthoxylates d'alcools gras, de préférence le produit de la réaction du dodécanol ou de l'alcool oléylique avec 1-5 unités oxyde d'éthylène.

14. Système thérapeutique transdermique selon l'une quelconque des revendications précédentes, **caractérisé en ce que** la matrice contenant le principe actif présente une teneur en au moins un stimulateur appartenant au groupe des esters d'acide gras formés avec le méthanol, l'éthanol ou l'isopropanol, de préférence le laurate de méthyle, l'oléate d'éthyle, le myristate d'isopropyle ou le palmitate d'isopropyle.

15. Système thérapeutique transdermique selon l'une quelconque des revendications précédentes, **caractérisé en ce que** la matrice contenant le principe actif présente une teneur en au moins un stimulateur appartenant au groupe des esters d'alcool gras formés avec l'acide acétique ou l'acide lactique, de préférence le lactate de lauryle ou l'acétate d'oléyle.

16. Système thérapeutique transdermique selon l'une quelconque des revendications précédentes, **caractérisé en ce que** la matrice contenant le principe actif présente une teneur en au moins un stimulateur miscible à l'eau appartenant au groupe comprenant les alcools aliphatiques à valences multiples ou les polyéthylèneglycols, de préférence le 1,2-propylèneglycol, le glycérol, le 1,3-butanediol, le dipropylèneglycol ou les polyéthylèneglycols ayant une masse moléculaire moyenne de 200 à 600 Da.

17. Système thérapeutique transdermique selon la revendication 16, **caractérisé en ce que** le stimulateur mentionné se trouve partiellement ou totalement sous la forme d'une émulsion dispersée dans la matrice, le stimulateur étant de préférence le glycérol.

18. Système thérapeutique transdermique selon l'une quelconque des revendications précédentes, **caractérisé en ce que** la matrice contenant le principe actif présente une teneur en au moins un plastifiant appartenant au groupe formé par les esters d'acide citrique ou les triglycérides saturés, de préférence le citrate de triéthyle, le citrate d'acétyltributyle, la triacétine ou les triglycérides qui présentent une longueur de chaîne moyenne, formés avec des acides gras ayant une longueur de chaîne de 8 à 12 atomes de carbone.

19. Système thérapeutique transdermique selon une ou plusieurs des revendications 10 à 18, **caractérisé en ce que** le stimulateur ou un mélange du stimulateur mentionné ou un mélange des stimulateurs mentionnés avec des stimulateurs non mentionnés constitue de 10 à 80 %, de préférence 10 à 50 % (m/m) de la matrice qui contient le principe actif.

20. Système thérapeutique transdermique selon l'une quelconque des revendications précédentes, **caractérisé en ce que** le système contient un agent solide adsorbant ou absorbant de l'eau à l'état sec, de préférence le sel de sodium ou de calcium de la carboxyméthylcellulose, la polyvinylpyrrolidone réticulée, le sel de sodium ou de calcium de l'acide polyacrylique ou polyméthacrylique réticulé et le sel de sodium ou de calcium du carboxyméthylamidon.

21. Système thérapeutique transdermique selon l'une quelconque des revendications précédentes, **caractérisé en ce qu'**il est prévu une couche séparée, adhésive sur sa face tournée vers la peau, constituée par un adhésif à base d'acrylate qui contient des groupes carboxyle, lesdits groupes carboxyle ne se trouvant toutefois pas sous forme de sel, et qui est réticulé de préférence par des ions aluminium.

22. Système thérapeutique transdermique selon l'une quelconque des revendications précédentes, **caractérisé en ce qu'**il est prévu une couche séparée, adhésive sur sa face tournée vers la peau, constituée par un adhésif à base d'acrylate qui est dépourvu de groupes carboxyle et contient des groupes hydroxyle, et qui est réticulé de préférence par des ions aluminium ou titane.

23. Système thérapeutique transdermique selon l'une quelconque des revendications précédentes, **caractérisé en ce qu'**il est prévu une couche séparée, adhésive sur sa face tournée vers la peau, constituée par un adhésif de fixation à base de silicone.

24. Système thérapeutique transdermique selon l'une quelconque des revendications précédentes, **caractérisé en ce qu'**il est prévu une couche séparée, adhésive sur sa face tournée vers la peau, constituée par un adhésif à base d'un mélange de polyisobutylènes qui présentent au moins 2 masses moléculaires moyennes différentes.

25. Système thérapeutique transdermique selon les revendications 21 à 24, **caractérisé en ce que** la couche qui est adhésive sur sa face tournée vers la peau présente un poids spécifique de 10 à 100 g/m², de préférence de 20 à 50 g/m².

26. Système thérapeutique transdermique selon l'une quelconque des revendications précédentes, **caractérisé en ce que** le principe actif pharmaceutique est la nicotine, la xanoméline ou la rivastigmine.

27. Système thérapeutique transdermique selon l'une quelconque des revendications précédentes, **caractérisé en ce que** le principe actif pharmaceutique est une hormone stéroïdienne, de préférence l'oestradiol, le lévonorgestrel, l'acétate de noréthistérone, le gestodène ou la testostérone.

28. Système thérapeutique transdermique selon l'une quelconque des revendications précédentes, **caractérisé en ce que** le principe actif pharmaceutique est un ester organique d'acide nitrique, de préférence la nitroglycérine, le mononitrate d'isosorbide ou le dinitrate d'isosorbide.
